# EUROPEAN PATENT APPLICATION

(11) **EP 4 679 436 A1**
(43) Date of publication of application: **14.01.2026**
(21) Application number: 24187821.4
(22) Date of filing: 10.07.2024
(51) Int. Cl.: G16H 10/40, G16H 40/20

(54) **SAMPLE PROCESSING METHOD**

(71) Applicant: Roche Diagnostics International AG, 6343 Rotkreuz (CH)
(72) Inventor: GLAUSER, Michael, 6343 Rotkreuz (CH); JANNER, Gabriele Piero, 6343 Rotkreuz (CH)
(74) Representative: Mewburn Ellis LLP

(57) **Abstract**

A computer-implemented method of processing a sample based on a digitized medical test order is provided. The computer-implemented method comprising the following steps: receiving, at a data processing agent, the digitized medical test order, the digitized medical test order comprising an image or a sound file comprising instructions for processing the sample; estimating, by the data processing agent, candidate text from the digitized medical test order by performing character recognition on the image or speech recognition on the sound file; comparing, by the data processing agent, the estimated candidate text to a set of predetermined test specifications to propose a candidate test order being requested by the digitized medical test order; and verifying, by the data processing agent, the candidate test order utilising context data associated with the digitized medical test order. When the candidate test order is successfully verified, the method comprises transmitting, by the data processing agent, the verified candidate test order to a control unit of an automated laboratory. The control unit is configured to match the sample to the verified candidate test order and instruct an automated analyzer of the automated laboratory to process said sample according to the verified candidate test order to obtain a test result.

## Description

### Field of the Invention

The present invention relates to a computer-implemented method of processing a sample based on a digitized medical test order.

### Background

An automated laboratory system is typically configured to receive a sample from a clinic for performing tests on according to instructions in a medical test order. The medical test order typically consists of several dimensions and lab parameters for defining which tests should be performed on the sample. For example, the medical test order may be provided to the automated laboratory in the form of industry standard test codes, such as Logical Observation Identifiers Names and Codes (LOINC) or codes from a specific laboratory catalogue, for specifying the requested tests.

However, a medical test order may be provided to the laboratory system by a user in a different format, e.g., from a clinic or physician, which needs translation into said industry standard test codes. For example, the medical test order may be provided as a hand-written note, a non-digital order form, text on paper order forms, or even as a recording of speech (e.g., in a dictation).

Such test orders require translation into standard test codes before being provided to an automated laboratory. Existing solutions for digitization of hand-written text or speech cannot be used for translating medical test orders owing to the hazardous impact of potential errors (e.g. on patient diagnosis or treatment). Additionally, a medical test order may consist of several dimensions (e.g., the 6 LOINC dimensions) and physicians may use different terms and synonyms for describing the same test which can make the translation of medical test orders particularly error prone.

Currently, the digitization and translation of medical test orders is performed manually by a person which is resource-intensive, error-prone, requires employee training, and can delay the processing of the sample, thereby delaying test results for patients. For example, [1] summarizes typical errors observed in central sample reception (CSR) involving humans.

Accordingly, there is a desire for a system which can more reliably translate a user provided medical test order into a test specification recognisable by a laboratory system. The present invention has been devised in light of the above considerations.

### Summary of the Invention

Broadly, the present invention relates to a method of parsing a medical test order and mapping the medical test order to an industry standard test specification. Importantly, the parsing and mapping of the medical test order is verified against context data associated with the medical test order to ensure that the parsing of the medical test order is correct.

Therefore, in a first aspect of the present invention there is provided a method of processing a digitized medical test order comprising the following steps: receiving the digitized medical test order, the digitized medical test order comprising an image or a sound file comprising instructions for processing a sample; estimating candidate text from the digitized medical test order; comparing the estimated candidate text to a set of predetermined test specifications to propose a candidate test order being requested by the digitized medical test order; and verifying the candidate test order utilising context data associated with the digitized medical test order.

Accordingly, by verifying the candidate test order using context data, the method of processing the test order is more reliable than existing speech-to-text and text recognition systems which do not verify the estimated text in a laboratory context.

The method may be a computer-implemented method. For example, the computer-implemented method may be performed by a data processing agent.

The method may form part of a method of processing a sample based on the digitized medical test order. For example, the method may further comprise processing a sample according to the candidate medical test order depending on the verification of the candidate medical test order. In this example, the method may further comprise: when the candidate test order is successfully verified, transmitting (e.g., by the data processing agent) the verified candidate test order to an automated laboratory for processing the sample according to the verified candidate test order. For example, the method may comprise: when the candidate test order is successfully verified, transmitting, by the data processing agent, the verified candidate test order to a control unit of the automated laboratory. Said control unit may be configured to match the sample to the verified candidate test order and instruct one or more automated analyzers of the automated laboratory to process said sample according to the verified candidate test order to obtain a test result.

The digitized medical test order may be instructions for processing the sample which are in a digital form (i.e., in a computer-readable format). For example, the digitized medical test order may comprise a digital scan or a digital image of a hardcopy medical test order. The hardcopy medical test order may be a handwritten medical test order. In other examples, the digitized medical test order may be an audio recording (i.e., a sound file) of a spoken medical test order. The digitized medical test order may not be computer-interpretable, in that it represents analogue information (e.g., an image or captured audio) which is subsequently used in a character recognition process.

In further examples, the digitized medical test order may include a plurality of, images, and/or a plurality of audio recordings. For example, the digitized medical test order may include a video file comprising multiple frames, each frame being an image. In further examples, multiple images of the same hardcopy test order or different parts of a hardcopy test order may be received.

The candidate text may therefore be estimated from the digitized medical test order by performing character recognition on the image(s) and/or speech recognition on the sound file(s). In each of these examples, the method of the first aspect provides more robust translation of the digitized medical test order into machine-recognisable instructions for the automated laboratory owing to the verification of the candidate test order against the context data.

The data processing agent may be one or more computer processors or server which is configured to receive and process digital files such as the digitized medical test order. For example, the data processing agent may be a remote server accessible over a network or located on a local computer, e.g., in a laboratory. The data processing agent may be a computer-implemented software module executed on one or more computing devices, such as a server, such that it is able to receive and process digital files such as the digitized medical test order. The data processing agent may be implemented on a single server, or multiple servers, and/or an internet-based cloud processing service such as Amazon AWS (RTM) or Microsoft Azure (RTM). The data processing agent, or a portion of it, may be hosted on a virtual machine.

The instructions for processing the sample may be a description and/or one or more test identifiers specifying one or more medical protocols or experiments to be performed on the sample.

The set of predetermined test specifications may be a plurality of names, indicators (e.g., abbreviations), or descriptions for industry standard tests. For example, the predetermined test specifications may be in the form of industry standard test codes for identifying industry standard tests, or descriptions, names, or abbreviations for known industry standard tests. For example, industry standard test codes may include codes defined by an organisation (e.g., LOINC), an IVD vendor, a laboratory, or a hospital, etc.

The candidate test order may include one or more of the industry standard tests specified in the set of predetermined test specifications which are estimated to be described in the digitized test order. The candidate test order may therefore be an initial estimate of the test order specified in the digitized test order which has not yet been verified using the context data.

The proposed candidate test order may be a selected one of the predetermined test specifications. For example, as mentioned above, the predetermined test specifications may include indicators or codes identifying industry standard tests. Thus, by comparing the candidate text to the indicators or codes of predetermined test specifications, the digitized medical test order can be mapped to one of the industry standard tests which is determined to be the candidate test order.

The verification of the candidate test order may include verifying the candidate test order as corresponding to a medical test which is requested on the digitized medical test order. The verification step may therefore ensure that the mapping of the digitized medical test order to an industry standard test is correct. The verifying of the candidate medical test order may also be referred to herein as validating the candidate medical test order.

The context data, described in more detail below, may include parameters or background information associated with a source of the digitized medical test order. The verifying of the candidate test order may therefore comprise determining if the candidate test order (or a test specified by the candidate test order) is consistent with and/or compatible with the background information. For example, the method may comprise determining an expected and/or historical behaviour of the source of the digitized medical test order based on the context data. The historical behaviour may include medical test orders which are commonly or previously requested from that source. The verifying may therefore comprise determining if the candidate test order is an expected test order based on the expected and/or historical behaviour of the source.

When the candidate test order is determined to correspond to the context data, the candidate medical test order may be considered as successfully verified. Determining that the candidate test order corresponds to the context data may include determining if the candidate test order is compatible and/or consistent with the context data. This may include determining if the candidate test order agrees with and/or matches one or more properties in the context data. For example, if there is a conflict between the candidate test order and the context data then it may be determined that the candidate test order is incompatible with the context data. Further, determining that the candidate test order corresponds to the context data may include determining, from the context data, if the candidate test order is a plausible candidate test order. This process is described in more detail below.

The verified medical test order may also be referred to as a validated test order or verified test specification. The verified medical test order may be formatted as one or more machine-recognisable test specifications. For example, the verified medical test order may comprise industry standard test definitions, e.g., using LOINC codes or a laboratory catalogue, which are recognisable by automated laboratories as defining a requested test.

The automated laboratory may refer to a system of one or more laboratory analyzers which are configured to perform one or more medical tests or protocols on a sample. For example, the medical tests or protocols may be for preparing, manipulating, or analysing the sample to measure one or more properties. Each of the analyzers may be configured to operate automatically to perform the one or more medical tests or protocols without manual input from an operator of the automated laboratory.

The control unit of the automated laboratory may be a computer or server which is configured to send computer-readable instructions to each of the laboratory analyzers, the computer-readable instructions being configured to cause the laboratory analyzers to process a sample according to those instructions. The control unit may be a computer implemented software module executed on one or more computing devices, such as a server, such that it is able to send instructions to the laboratory analyzers. The control unit may be implemented on a single server, or multiple servers, and/or an internet-based cloud computing processing service such as Amazon AWS (RTM) or Microsoft Azure (RTM). The control unit, or a portion of it, may be hosted on a virtual machine. The control unit may be in communication with the data processing agent. The control unit may receive computer-readable instructions from the data processing agent including one or more medical test orders, for example as standard test codes, which specify one or more tests for processing samples using the laboratory analyzers. Thus, the control unit may be configured to process the computer-readable instructions from the data processing agent and instruct the one or more laboratory analyzers to process the sample according to the instructions.

The method may comprise retrieving and/or receiving context data associated with the digitized medical test order. Receiving the context data can be performed at any point in the method prior to verifying the candidate medical test order. For example, the context data may be received with the digitized medical test order. That is, a message including the digitized medical test order, received by the data processing agent, may also include the associated context data and/or an identifier for use in retrieving the context data e.g., from a database. In other examples, the digitized medical test order itself may comprise context data. In further examples, some or all of the context data may be received from a Hospital Information System (HIS) and/or a Laboratory Information System (LIS). The context data may be static data or may be dynamic data, and further, may be held by the entity performing the validation/verification. For example, the data processing agent may retain a database in which context data relating to the originator of the digitized medical test order is held.

When, or if, the candidate test order is not successfully verified, the method may further comprise rejecting the digitized medical test order. For example, the candidate medical test order may be determined to be incompatible with some or all of the context data. This may potentially indicate that the estimated text contains an error or that the proposed candidate medical test order derived from the estimated text is incorrect.

Rejecting the digitized medical test order (or the candidate medical test order) may comprise generating a message and/or an indication for a user interface that the digitized medical test order is rejected. For example, a communication to a user (e.g., physician or a patient) that submitted the digitized medical test order may be generated indicating that the digitized medical test order has been rejected. In some examples, a user may be prompted to perform a manual/visual check of the digitized medical test order against the rejected candidate test order to override the rejection or to select an alternative verified medical test order which correctly corresponds to the digitized medical test order.

In further examples, when the digitized medical test order is rejected, the method may comprise obtaining clarification information from the user. For example, the user may be a lab operator or a user who originally provided the digitized medical test order (e.g., a physician). Accordingly, the user may provide clarification information describing the medical test order, provide additional context data, and/or indicate a correct medical test order which was requested in the digitized medical test order.

In some examples, when or if the medical test order is rejected, the method may further comprise generating one or more alternative candidate test orders. The one or more alternative candidate medical test orders may be generated based on the context data and/or the estimated candidate text. For example, the one or more alternative candidate test orders may be determined (e.g., by an ML algorithm) to be a next most likely candidate medical test order(s) according to the context data and the estimated text. An indication of the one or more alternative candidate medical test orders may generated and, optionally, communicated to a user or provided to a user interface. Where there is a plurality of alternative candidate medical test orders, they may be provided to the user ordered by relevance.

As mentioned above, the digitized medical test order may be a digital image of a hardcopy test order. The method may further comprise scanning or receiving a scan of the hardcopy of the medical test order to receive the digitized test order. For example, the hardcopy may be a handwritten test order. In other examples, the hardcopy may comprise stamped, printed or typed text forming the medical test order which may be digitally scanned. Determining the candidate text may comprise performing machine learning (ML) based text recognition on the digitized medical test order.

In some examples, the digitized medical test order may include multiple digital images or video frames, e.g., depicting one or more hardcopy test orders. In these examples, the method may comprise estimating respective candidate text from each of the candidate images and combining the respective candidate text. In other examples, the method may comprise determining a selected one of the multiple images with the highest quality, for example the sharpest image, and then estimating the candidate text from the selected one of the multiple images.

The set of predetermined test specifications may have associated synonyms and/or test parameters or properties. For example, the predetermined test specifications may be stored in a database, e.g. under test names and or industry codes, wherein the predetermined test specifications are stored in association with alternative names or parameters/options associated with the respective test. Comparing the estimated candidate text to the set of predetermined test specifications may comprise comparing the estimated candidate text to the associated synonyms and test parameters of each predetermined test specification.

Proposing the candidate test order may comprise determining a subset of the predetermined test specifications with associated synonyms and test parameters representing the closest matches to the estimated candidate text, and selecting a most likely of the predetermined test specifications in the subset as the candidate test order. For example, selecting the most likely of the predetermined test specifications in the subset may include excluding predetermined test specifications in the subset which have associated properties which are not included in the estimated candidate text. For example, if a first test is associated with the property: "high sensitivity," then this test specification may be excluded from being selected unless the candidate text includes the words "high sensitivity."

In some examples, selecting the most likely of the predetermined test specifications in the subset may include, for each of the predetermined test specifications in the subset, determining if the predetermined test specification is compatible with the context data associated with the digitized medical test order, and excluding predetermined test specifications in the subset which are not compatible with the context data.

The set of predetermined test specifications may be formatted as industry test codes having associated test parameters or properties. Proposing the candidate test order may therefore comprise mapping the estimated candidate text to one of the industry test codes. For example, the industry test codes may be Logical Observation Names and Codes (LOINC) codes.

The context data may comprise one or more from a group containing: patient data, patient test order history, patient demographic information, clinic location of origin of the digitized medical test order, clinic test order history, target laboratory for the digitized medical test order, target laboratory test availability, identity of a physician submitting the medical test order, sample type associated with the digitized medical test order, and/or additional digitized medical test orders submitted in combination with the digitized medical test order. Some of the context data, e.g. Order ID, Patient ID, Sample ID, may be obtained by scanning a barcode or QR code, wherein said barcode or QR code may be part of the digitized test order.

For example, each of the above examples of context data may have associated properties against which the candidate medical test order may be checked against. For example, clinic location may be a source location of the digitized medical test order and may be associated with a particular medical specialism, that medical specialism being associated with a plurality of medical tests. Therefore, if the candidate medical test order is not included in the plurality of medical test associated with that clinic, then the candidate medical test order may be rejected as being unlikely to have originated from that clinic.

Additional digitized medical test orders submitted in combination with the digitized medical test order may be compared to known sequences of test orders which are commonly ordered together (e.g., to diagnose a particular condition). Therefore, if a plurality of candidate medical test orders are generated from a respective plurality of digitized medical test orders, each of the plurality of digitized medical test orders being associated with a single patient, which are not commonly ordered together, then one or more of the candidate test orders may be rejected as being unlikely to be correct.

In some examples, the context data may comprise a target laboratory location associated with the digitized medical test order such as a clinic and available medical tests provided by that laboratory. If the candidate medical test order relates to a medical test which is not provided by the target laboratory, then the candidate medical test order may be rejected as unlikely to be correct.

Verifying the candidate test order may comprises performing a binary determination of whether the candidate test order is compatible or not with the context data. The digitized medical test order may be rejected if the candidate test order is incompatible.

In some examples, verifying the candidate medical test order may include using the context data to determine a likelihood of the candidate test order being correct. The likelihood may be compared to a configurable threshold. The candidate medical test order may then be successfully verified or rejected depending on if the determined likelihood is above the threshold. For example, the candidate medical test order and the context data associated with the digitized medical test order may be provided to a trained machine learning model as inputs, the machine learning model being configured to output a likelihood of the candidate test order being correct based on the context data. In this context, the candidate medical test order being "correct" may be considered to mean that the candidate medical test order corresponds to what is requested in the digitized medical test order (e.g., the candidate medical test order is what is written on the digitized medical test order or recited in a recorded speech).

For example, when the context data may comprise clinic and/or patient history information (the clinic being a source location of the digitized medical test order), verifying the candidate test order may comprise determining a likelihood of the candidate medical test order being requested (i.e., being correct) based on the clinic and/or patient history information.

In further examples, the context data may comprise a clinic and/or physician medical specialism(s) associated with the digitized test order (the clinic and/or physician being a source of the digitized medical test order), wherein verifying the candidate test order may comprise determining a likelihood of the candidate test order being requested in relation to that medical specialism.

Verifying the candidate test order may comprise: retrieving test parameters associated with the candidate test order, comparing the test parameters to the context data, and determining if the context data corresponds to the test parameters. Determining if the context data corresponds to the test parameters may comprise performing a binary determination of whether the context data is compatible with the test parameters, wherein if the context data is compatible with the test parameters, the candidate test order is successfully verified.

For example, the test parameters may include an expected patient demographic associated with the candidate test order. In this example, the context data may include patient information (of a patient from whom the sample was provided), the patient information including patient demographic data. Verifying the candidate test order may therefore comprise determining if the patient demographic data corresponds to the expected patient demographics. For example, the patient demographic data may comprise a patient age or age range of a patient associated with the digitized test order. The expected patient demographics may comprise an approved age range(s) associated with the candidate test order. Therefore, verifying the candidate test order may comprise determining if the patient age is included in the approved age range(s).

In further examples, the context data and/or the estimated candidate text may comprise diagnosis information such as a suspected diagnosis or diagnosis query. The test parameters of the candidate medical test order may comprise suspected diagnoses related that test. Accordingly, verifying the candidate medical test order may comprise determining a likelihood of that candidate medical test order being requested in relation to the diagnosis information in the context data.

In some examples, if the context data is determined to be incompatible with one or more of the test parameters, the method may further comprise retrieving priority information associated with the test parameter determined to be incompatible, and re-determining the likelihood of the candidate test being correct based on the context data and the priority information. The priority information may be an indication or importance and/or relevance of the test parameter for the test specification. For example, the test parameter may be indicated to be a highly relevance/high priority test parameter or a low relevance/low priority test parameter. For example, if test parameter indicates that the test may only be performed on a blood sample, this may be indicated to be a high priority test parameter. Therefore, if the context data indicates that the sample is not blood, the candidate test order may be rejected. However, if the test parameter is a preferred patient age range, this may be indicated to be a low priority test parameter which is less likely to affect the likelihood of the candidate test being correct.

In further examples, the context data may include a priority level of the digitized medical test order. Accordingly, if a digitized medical test order has a high priority level, and the candidate test order is rejected, the computer-implemented method may comprise transmitting the rejected candidate test order to the control unit of the automated laboratory even though it was rejected. The method in such instances may also include transmitting a message to the originator of the medical test order indicating that the candidate test order was not successfully verified but is still being processed. Alternatively, if a digitized medical test order has a low priority level, and the candidate test order is rejected, the computer-implemented method may comprise requesting human verification of the digitized test order or a second digitized medical test order to be provided.

In a second aspect of the present invention, there is provided a data processing agent for processing a digitized medical test order, configured to perform any of the methods of the first aspect. For example, the data processing agent comprises: a test order input module configured to receive a digitized medical test order, the digitized medical test order comprising an image or a sound file comprising instructions for processing a sample, a text generation module configured to estimate candidate text from the digitized medical test order by performing character recognition on the image or speech recognition on the sound file; a text comparison module configured to compare the estimated candidate text to a set of predetermined test specifications to propose a candidate test order being requested by the digitized test order; and an order verification module configured to verify the candidate test order utilising context data associated with the digitized medical test order; and verify the candidate test order utilising context data associated with the digitized medical test order; and an instruction transmission module configured to, when the candidate test order is successfully verified, transmit the verified candidate test order to a control unit of an automated laboratory for instructing an automated analyzer to process said sample according to the verified candidate test order.

The data processing agent may therefore be referred to as a test order input unit. The data processing agent may further comprise a database comprising the set of predetermined test specifications and associated test parameters and/or synonyms for each of the predetermined test specifications.

The data processing agent may further comprise or be communicatively coupled to a user control module. The data processing agent may further comprise or be communicatively coupled to a digital scanner and/or a camera. One or more of the modules of the data processing agent may be a cloud-based module. For example, the digitized medical test order may be received at a local device from a user and communicated to the data input module for processing on a cloud server.

In an additional aspect of the present invention there is provided an automated sample processing system comprising a data processing agent, and one or more automated laboratories, each automated laboratory comprising a control unit communicatively coupled to one or more analysers of the automated laboratory. The data processing agent may be the data processing agent of the second aspect. The control unit may be configured to receive the verified candidate medical test order received from the data processing unit, match a sample to the verified candidate medical test order, and instructs one of the automated analyzers to process the sample according to the verified candidate test order to obtain a test result.

The data processing agent may be configured to communicate with the control unit of one or more automated laboratories via the cloud.

In a further aspect, there is provided a computer program product comprising instructions which when executed by a computer cause the computer to perform the method of the first aspect and any optional features thereof. In a further aspect, there is provided a computer-readable storage medium, having stored thereon, the computer program of the previous aspect.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Fig. 1** shows a high-level system diagram of an automated laboratory system;
**Fig. 2** shows a data processing agent of the automated laboratory system of Fig.1;
**Fig. 3** shows a flow diagram of a process for processing a medical test order comprising instructions for processing a sample according to aspects of the present invention; and
**Fig. 4** shows an example database of LOINC test codes and test properties.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

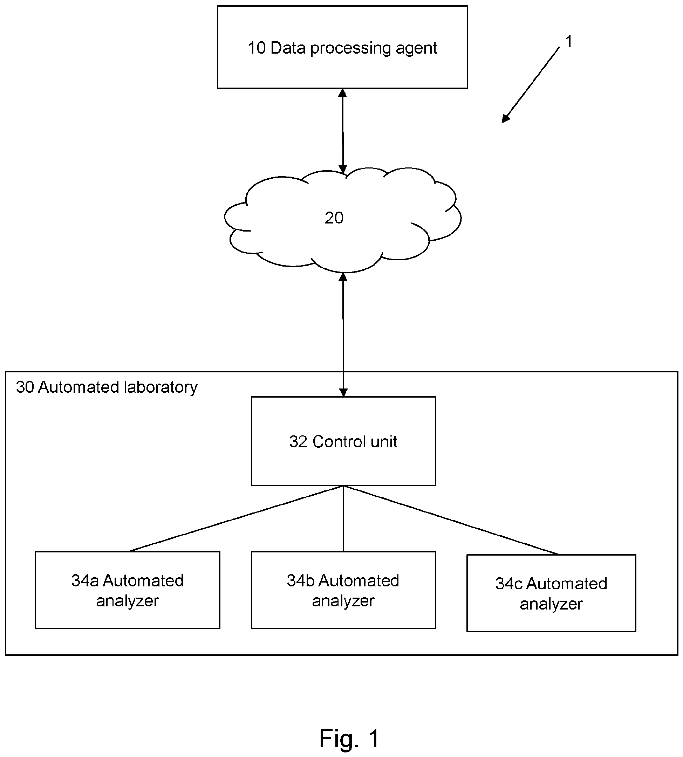

Fig. 1 shows a high-level system diagram of an automated laboratory system 1 for processing a biological sample according to instructions provided in a medical test order. The system of Fig.1 may be employed to execute computer-implemented methods according to aspects of the present invention.

The automated laboratory system 1 comprises a data processing agent 10 for receiving and processing a medical test order, and one or more automated laboratories 30 for performing tests on biological samples according to test specifications received from the data processing agent 10. In this example, the data processing agent 10 is in communication with a control unit 32 of the one or more automated laboratories 30 via the cloud 20. The one or more automated laboratories 30 contain a plurality of automated analyzers 34a-c which are configured to perform tests on the biological sample according to instructions received from the control unit 32.

To process a sample using the automated laboratory system 1, a digitized medical test order is first generated by a user and provided to the data processing agent 10. For example, the user may be a physician, associated with a clinic, a GP surgery, or a telemedicine unit and the digitized medical test order may be digital scan of a filled in order form or a handwritten test order, or an audio recording of a speech.

In addition to the medical test order, a sample is obtained from a patient and provided to one of the automated laboratories 30 for testing. The analog medical test order contains instructions and/or specifications for tests to be performed on that sample by one of the automated laboratories 30.

The data processing agent 10 is configured to translate the digitized test order into a verified machine-recognisable/readable test order which is recognisable by the automated laboratory system 1. For example, the verified test order may be in the form of industry standard test codes, test specifications, or machine executable instructions.

The data processing model 10 is configured to provide the verified test order containing machine recognisable instructions to the control unit 32 of one of the automated laboratories 30 e.g., via a network connection (e.g., via a wide area network connection, such as the cloud 20).

The control unit 32 is configured to match the verified test order to an associated biological sample from the patient and instruct one of the automated analyzers 34a-c to process the sample according to the tests specified in the verified test order.

The data processing agent 10 may be located on a local system such as a terminal or computer which is accessible to the user. Alternatively, the data processing agent 10 may be located on a remote server or in middleware of the automated laboratory system 1. In some examples, the functionality of the data processing agent 10 may be divided between systems. For example, character recognition (described below) to parse the digitized test order may be performed locally on a user terminal, whereas translation of the resulting text into a machine recognisable verified test order may be performed remotely (e.g., in the cloud).

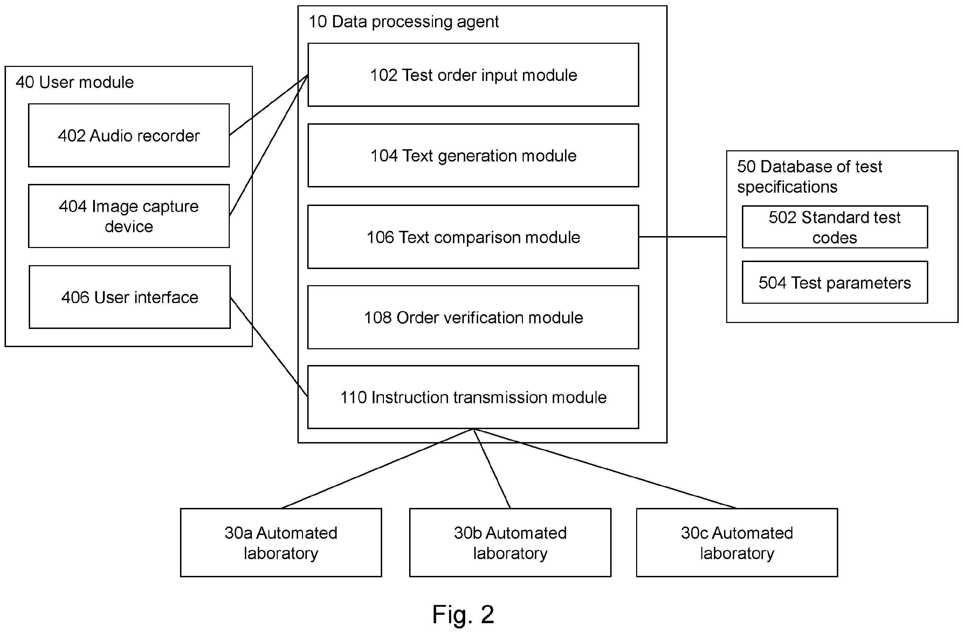

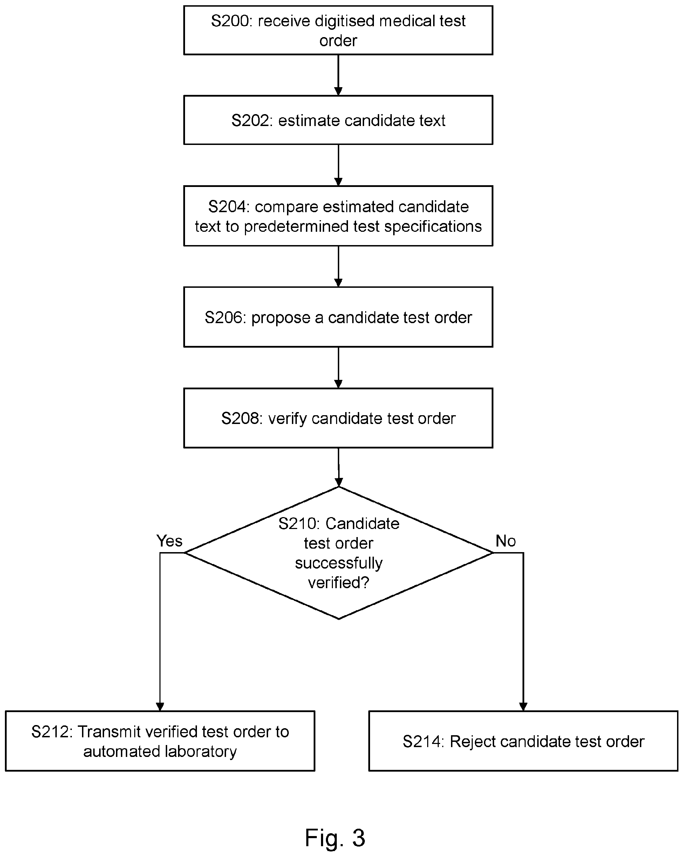

Importantly, after translating the digitized test order into a machine-readable test order, the data processing agent 10 is configured to verify the machine-readable test order to ensure that the translation was correct, i.e., to ensure that the tests specified by the machine-readable test order correctly reflect the tests ordered in the digitized test order. Before verification, the machine-readable test order may be referred to as a candidate test order. The candidate test order is verified utilising context data associated with the digitized medical test order as discussed below in relation to Figs. 2 and 3.

Fig. 2 shows a more detailed diagram of the data processing agent 10 of the automated laboratory system 1.

The data processing agent 10 comprises a test order input module 102 for receiving the digitized medical test order, a text generation module 104 for proposing estimated text from the digitized test order, a text comparison module 106 for comparing the estimated text to a set of predetermined test specifications to propose a candidate test order, an order verification module 108 for verifying the candidate test order, and an instruction transmission module 110 for transmitting the verified candidate test order to an automated laboratory 30.

Herein, the term "module" is used to refer to a functional module which is configured or adapted to execute a particular function. The modules may be implemented in hardware (i.e. they may be separate physical components within a computer), in software (i.e., they may represent separate sections of code, which when executed by a processor, cause the processor to perform a particular function), or in a combination of both.

In this example, the automated laboratory system 1 comprises a plurality of automated laboratories 30a-c, each configured to receive verified (machine-readable) medical test orders from the data processing agent. Each digitized medical test order provided to the data processing agent 10 may have an associated target laboratory for processing the sample. Alternatively, the data processing agent 10 may be configured to automatically assign the medical test order to one of the automated laboratories 30a-c depending on the tests that are specified in the test order.

The test order input module 102 is configured to receive the digitized medical test order in the form of one or more digital images or audio files as discussed above. In this example, a user module 40 in communication with the data processing agent 10 is provided to facilitate user interaction with the data processing agent 10. The user module 40 may be a user terminal or a computer located, for example in a remote patient clinic, in a physician's office, or in a laboratory, etc.

The user module 40 comprises a user interface 406 for displaying information to a user and for receiving user input. For example, the user interface 406 may include a touchscreen, a display showing a GUI (Graphical User Interface), a mouse, a keyboard, etc. In some examples, when the user module is located in a laboratory, the user module 40 may include a laboratory operator interface for enabling an operator of the laboratory to configure and monitor the laboratory.

Additionally, the user module 40 comprises an audio receiver 402, e.g., a microphone, for recording speech from the user. Additionally, the user module 40 comprises an image capture device 404, e.g., a camera or digital scanner, for digitising a hardcopy of a written medical test order. The test order input module 102 is thus configured to receive the digitized medical test order from the audio recorder 402 in the form of an audio file, or from the image capture device 404 e.g., in the form of a digital image or PDF. In some embodiments, the user module 40 may contain only one of the audio receiver 402 and the image capture device 404.

The text generation module 104 is configured to receive a digital file containing the digitized medical test order from the test order input module 102 and generate estimated text which is based on the writing or speech in the digitized medical test order. For example, when the digitized medical test order contains an image, the text generation module 104 is configured to perform optical character recognition on the digitized medical test order to estimate the candidate text. When the digitized medical test order is an audio file, the text generation module 104 is configured to perform automated speech-to-text recognition on the sound file to generate the estimated text. As discussed below in relation to Fig. 3, the optical character recognition (OCR) or speech-to-text recognition may be performed using an off-the-shelf machine learning algorithm. Alternatively, a bespoke machine learning algorithm may be trained e.g., using context specific laboratory data.

The text comparison module 106 is configured to receive the estimated text from the text generation module 104 and compare the estimated text to a set of predetermined test specifications to propose a candidate test order being requested by the digitized test order. The candidate test order contains standard test codes or machine-readable instructions for instructing the automated analyzers 34a-c of the automated laboratory as discussed above. In this example, the text comparison module 106 is communicatively coupled to a database of predetermined test specifications 50. For example, the database may be accessible via the internet, from local storage, or via an intranet.

In this example, the database 50 contains tests represented by standard test codes 502 (e.g. industry standard codes such as LOINC codes or IVD vendor codes, or other standard test codes such as order codes from specific laboratory catalogues) which specify particular biomedical tests which may be performed by the automated laboratories 30. The database 50 also contains test parameters associated with each test and its respective standard test code. By comparing the estimated text written on the medical test order to the test parameters 504 in the database, the text comparison module 106 is able to determine a most likely test that was requested on the medical test order and retrieve the associated standard test codes for that test. The resulting standard test codes therefore form a candidate (machine-readable) test order.

The order verification module 108 is configured to retrieve context data associated with the digitized medical test order and verify the candidate (machine-readable) test order received from the text comparison module 106 using the context data to verify that that candidate test order corresponds to the original digitized test order provided by the user. After successful verification by the order verification module 108, the candidate medical test order may be referred to as a verified candidate medical test order.

The order verification module 108 is configured to provide the verified candidate medical test order to the instruction transmission module 110 which, in-turn, is configured to transmit the verified medical test order to one of the automated laboratories 30 for testing of the sample. For example, the verified medical test order may be communicated to the control unit, for example, over the internet, via a local wired or wireless network, or using local storage (e.g., using a memory stick or CD).

Additionally, the instruction transmission module 110 may transmit the verified test order to the user interface 406 for displaying to a user. In examples, where the order verification module 108 rejects a candidate test order, then the instruction transmission module 110 may transmit a notification to the user interface 406 that the candidate test order has been rejected. The user may use the user interface 406 to provide clarification information, provide additional context data, or suggest a different test order, etc.

Fig. 3 shows a flow diagram of a process for processing a medical test order. The process of Fig. 1 may be a computer-implemented method performed by the processing agent 10 of Fig. 1.

First, in step S200 the processing agent receives a digitized medical test order.

The digitized medical test order may be a digital copy of an analog test order. For example, an analog medical test order may be generated by a user (i.e., a test orderer) as a hardcopy of an order form on paper or a speech recording. The method may then comprise an additional step of digitising the analog medical test order. For example, a hardcopy medical test order may be digitized using a scanner, or a camera to generate a picture or PDF of the hardcopy medical test order.

In some examples, digitisation of the test order may not be necessary. For example, if the medical test order was handwritten directly into an electronic system e.g., using a stylus and a tablet computer. Alternatively, the digitized test order may be a digital copy of an audio file containing speech.

In step S202, the data processing agent 10 performs optical character recognition (OCR) on the digitized test order to estimate candidate text. Any other suitable processing to translate printed or handwritten text to machine recognisable text may also be used to perform this step.

For example, this step may include text processing using machine learning which comprises applying a trained model to the digitized image of the paper order/label/form containing printed text and/or human-written text, or to an audio recording. For example, an existing text or speech recognition engine may be used such as: MetaMoJi Note, Microsoft OneNote, MyScript Nebo, Notes Plus, Mazec, Pen to Print, Notability, Gboard with Google handwriting input, Pyimagesearch, OpenCV, Handwriting Recognize (Chrome), or GoodNotes 5.

Alternatively, the estimated text may be generated using a custom trained model. Training of the custom model may be performed using a dataset with historical test/speech samples which may be: generic (i.e., not specific to laboratories / IVD (In Vitro Diagnostics)) such as Kaggle A-Z dataset¹; MINIST Dataset²; specific to medical, laboratory, IVD, EHR fields, such as the list of available tests in a lab and in standards as LOINC; specific to a person (physician, nurse, etc); contain scans of paper documents such as laboratory orders, sample labels, or paper forms; and/or specific to a language, region, laboratory chain or it may cover multiple languages or regions. Furthermore, the training data may be labelled and thus associated with (part of the) contained text in digital form.
¹ https://www.kaggle.com/datasets/sachinpatel21/az-handwritten-alphabets-in-csv-format
² https://github.com/sharmaroshan/MNIST-Dataset

Training of the custom model may involve one of or a combination of: supervised learning e.g. using labelled data as classification and regression techniques; unsupervised learning e.g. using non-labelled data as clustering and association techniques; semi-supervised learning e.g. using both labelled and non-labelled data as classification and clustering techniques; or reinforcement learning e.g. with rewards. The methods of training and/or the machine learning model type may include one or a combination of: Classification: Naive Bayes (NB), Linear Discriminant Analysis (LDA), Logistic regression (LR), K-nearest neighbours (KNN), Support vector machine (SVM), Decision tree (DT), Random forest (RF),Adaptive Boosting (AdaBoost), Extreme gradient boosting, (XGBoost), Stochastic gradient descent (SGD), Rule-based classification; Regression Analysis (Simple and multiple linear regression, Polynomial regression, LASSO and ridge regression); Clustering: Partitioning methods, Density-based methods, Hierarchical-based methods, Grid-based methods, Model-based methods, Constraint-based methods, K-means clustering, Mean-shift clustering, Density-based spatial clustering of applications with noise (DBSCAN), Gaussian mixture models (GMMs) clustering, Agglomerative hierarchical clustering; Dimensionality Reduction and Feature Learning: Feature selection, Feature extraction, Variance threshold, Pearson correlation, Analysis of variance (ANOVA), Chi square statistic, Recursive feature elimination (RFE), Model-based selection, Principal component analysis (PCA); Association Rule Learning: AIS and SETM, Apriori, Equivalence Class Clustering and bottom-up Lattice Traversal (ECLAT), Frequent-Pattern tree Growth (FP- Growth), ABC-RuleMiner; Reinforcement Learning: Monte Carlo methods, Q- learning, Deep Q-learning; Artificial Neural Network (ANN) and Deep Learning: Multi-layer Perceptron (MLP), Convolutional Neural Network (CNN, or ConvNet), Long Short-Term Memory Recurrent Neural Network (LSTM-RNN). Libraries and frameworks for training the custom model include: Keras, TensorFlow, Microsoft Azure ML Studio, OpenAl, PyTorch, IBM Watson ML, and Chainer.

In step S204, the estimated candidate test is compared to predetermined test specifications in order to translate test requested on the digitized test order to a candidate test order containing machine-readable lab parameters. For example, the machine-readable lab parameters may be standard test codes such as LOINC codes, or IVD vendor codes such as Roche Application Code Number (ACN).

In some examples, the estimated candidate text may be compared to test properties associated with each test specification which is represented by standard test codes in order to determine a most likely test being requested in the digitized medical test order.

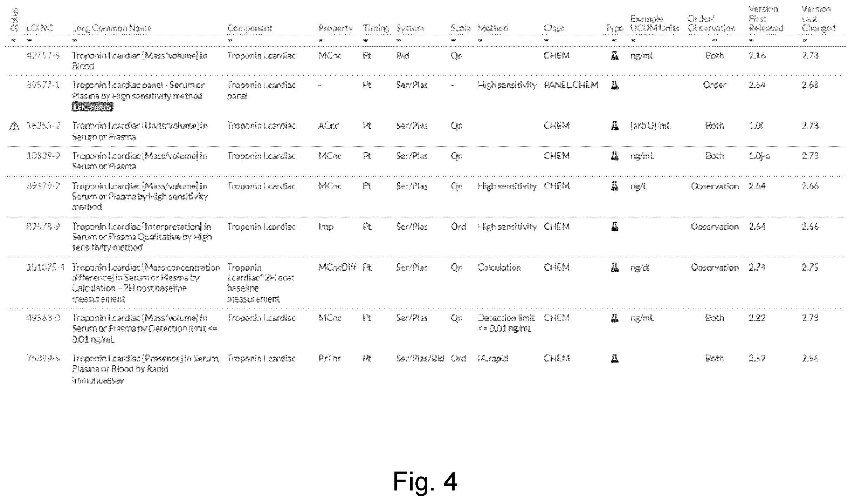

For example, to perform LOINC code mapping, a database is accessed comprising standard test codes and possible synonyms associated with each standard test code. An example of such a database comprising LOINC codes is shown in Fig. 4. Accessing the database may include using asterisks (*), which may represent any value, and/or regular expressions as used in standard data processing steps. For example, the regular expressions may include specific text syntax which represent text patterns for a text search of the database.

As an example, the estimated text determined in step S202 may include 'Troponin I concentration' where the sample material is serum. In consulting the database of standard test codes, by comparing the estimated text to the synonyms and test properties, the data processing agent 10 proposes a subset of the standard test codes having the closest matches (of synonyms and test parameters) to the estimated text. The rows of the database containing the closest matches are shown in Fig. 4 for 'Troponin I concentration' and 'serum'.

Next, the data processing agent 10 selects a most likely one of the subset of standard test codes as a candidate test order. Selecting the most likely of the LOINC code may include excluding codes which have certain associated properties which are not included in the estimated candidate text. For example, the data processing agent 10 may exclude all rows of the table of Fig. 4 directed to "high sensitivity codes" (indicated in the "method" column) because the digitized test order did not specify a "high sensitivity" test. Additionally, the row having PrThr (meaning "Presence or Threshold") in the "property" column may be excluded because this test would provide a binary result rather than a concentration as required in the estimated text ("Troponin I concentration").

Next, additional rows may be excluded by comparing the sample type obtained from the patient with an expected sample time for each test. Therefore, in this example, row 42757-5 is excluded because the associated sample type is blood as opposed to serum. Additionally, in this example, 16255-2 may be excluded due to the presence of status 'discouraged' (indicated by the triangular warning symbol) indicating that this test is unlikely to have been requested on the medical test order and because of the presence of ACnc (meaning "Arbitrary concentration") in the property column. The only remaining match (10839-9) in Fig. 4 is for serum and plasma (system=Ser/Plas) and therefore is determined to be the most likely of the tests.

In step S206, the data processing agent 10 proposes the most likely of the test specifications (10839-9 in this example) as a candidate test order.

The data processing agent 10 may perform an additional step of mapping the candidate test order to a machine-readable format which is compatible with the automated analyzers 34a-c of the automated laboratory 30. This format may be configured depending on an IVD vendor associated with the automated laboratory 30. For example, LOINC codes may be mapped to manufacturer specific test identifiers (for example Roche ACN (Roche Application Number) codes).

In one example, for the Glucose HK Gen.3 assay from Roche Diagnostics (PN: 08057800190) with serum/plasma as sample there is one possible ACN code listed: 20630. The mapping shows two possible LOINC Terms for cobas c 503 instrument: 2345-7 with unit mg/dL or 14749-6 with unit mmol/L. To decide between these two options, the standard settings in the automated laboratory 30 may be consulted, e.g. installed parameters and their units. Alternatively, in some examples, direct mapping from the estimated text to ACN without the LOINC code is possible.

Next, in step S208 the data processing agent 10 verifies the proposed candidate test order as corresponding to the requested test on the digitized medical test order. The verification is performed by comparing context data associated with the digitized medical test order to known test parameters associated with the candidate test order.

In step, S210 the data processing agent determines whether the candidate test order is successfully verified or not depending on if the candidate test order is determined to be a likely test order in the present context and if the context data and the candidate test order are compatible. If the candidate test order is not successfully verified, then the data processing agent 10 moves to step S214 and rejects the candidate test order. However, if the candidate test order is successfully verified, then the data processing agent 10 moves to step S212 and transmits the verified candidate test order to the control unit of an automated laboratory 30 which is configured to processing the sample according to the received test order.

The following sections describe examples of context data and methods for verifying the candidate medical test order. The verification method may include any combination of the following examples, for example depending on the context data that is available to the data processing agent.

In some examples, the context data may include patient history and/or information from a clinical decision support system (CDSS). For example, the context data may include regularly ordered tests (e.g. periodic HbA1c status). The verification step may then take into account:
i. If a patient has regular scheduled blood tests taken, e.g. HbA1c status during Glycemic control of diabetes, in minimum recommended twice a year and the testing time point is within the usual time frame for a next test, the verification takes this high probability into account.
ii. If a patient has regular therapeutic drug monitoring (TDM) tests measured, there can also be a median / average testing interval deduced from the CDSS and verification takes those high probability tests into account, like monitoring the serum concentration of lithium. For example, more tests can be found in chapter 18 of [2].
iii. If a patient has an autoimmune diseases (see chapter 25 in [2]) the immune system needs to be periodically surveyed to determine e.g. complete blood count (CBC). This test can be indicated by the data processing agent 10 as a test with a high probability of being ordered for that patient.

In further examples, the context data may include information about clinical pathways, order history and/or test results which may result in particular follow up tests being ordered.

For example, in hyperthyroidism according to [2], if a previous TSH (Thyroid Stimulating Hormone) test shows lowered values of TSH, then there is a high probability that a subsequent test order may include tests for fT4 (free thyroxine) and fT3 (free triiodothyronine). Therefore, the verification of the subsequent test order would be successful when a candidate test order includes tests for fT4 and fT3. In some examples, there may also be a suspected diagnosis (hypothesis) written on the order form, which can be considered for the verification.

Furthermore, in this example, if a sequence of test orders for a patient resulted in 'TSH lowered values, fT4 and or fT3 elevated' and a next test order is incoming, then the tests for TSHR Ab, TPO Ab, and Tg Ab, may each be classified as having a high probability of being included in the next test order. Therefore, if the candidate test order contains TSHR Ab, TPO Ab, or Tg Ab, then the verification may be successful based on the previous sequence of test orders. More clinical pathways can be found in the respective medical literature, for example in [3] which presents several examples of clinical pathways.

Thus, in these examples, the verification of the candidate test order includes using a ML/AI algorithm to determine a next most likely test being ordered based on patient history.

For example, LIS and/or EMR data may be used for the training of an AI/ML model wherein the model is given patient data up to a certain time point (the patient data including any combination of: patient demographics, past diagnoses, other ordered tests, test results, treatments, geographic location, ordering physician, healthcare institutions, laboratory, notes, images, etc.). The model is therefore trained to predict next test(s) ordered. For supervised training, each patient's data can be split into two parts by selecting a timestamp: all the entries before the timestamp compose the historical data and the ones afterwards are the future data to be learned by the model.

Additionally, the context data may comprise patient demographics, such as gender or date of birth, in order to determine tests that are very unlikely (or never) ordered. For example, test orders which are unlikely (and so are unlikely to be successfully verified) may include:
i. newborn patient should be tested on fertility test (ovarian reserve and the prediction of response to controlled ovarian stimulation) with anti-Müllerian hormone (AMH) test
ii. 80 year old patient should be tested with the newborn screening program³ (blood spot test) on 9 rare conditions (e.g. sickle cell disease, cystic fibrosis, congenital hypothyroidism, inherited metabolic diseases)
³ https://www.nhs.uk/conditions/baby/newborn-screening/blood-spot-test/

Furthermore, the context data may include medical department and/or physician specialty that ordered the test. This information may be used to exclude (or indicate as very unlikely) some tests (based on the test itself or on its attributes as e.g.: sample type required, time to result). For example, unlikely orders may be:
i. A cardiac specialist ordering cancer tests (Elecsys CA 15-3 II) for the aid in the management of breast cancer.
ii. An emergency department ordering a fungal culture test (needing multiple days until result)
iii. A dentist ordering a test only performable on cerebrospinal fluid for Alzheimer's disease (Elecsys Total-Tau CSF)

In further examples, the context data may include a suspected diagnosis. Accordingly, the data processing agent 10 can compare the suspected diagnosis to a disease area group associated with the candidate test order. If the suspected diagnosis and the disease area group are incompatible, then the candidate test order may be rejected and an alternative test order may be proposed. In this example, the context data may be retrieved from a database comprising disease or diagnosis area groups, suspected diagnoses and corresponding lab parameters. Examples, of tests and their associated disease areas are listed below compared to different tests which have similar identifiers but are from different disease areas:
i. HCV (Infectious Diseases) and hCG (Fertility)
ii. LDH (Hepatology/Coag.) and HBDH (Endocrinology/Cardiac)
iii. THC (DAT) and TSH (Endocrinology)
iv. TSH (Endocrinology) and FSH (fertility)
v. Further possible mismatches can also be found in the literature⁴
⁴ Automated misspelling detection and correction in clinical free-text records, Kenneth H. Lai et al, Journal of Biomedical Informatics, Volume 55, June 2015, Pages 188-195, https://doi.org/10.1016/j.jbi.2015.04.008

In these examples, the tests differ from each other by one letter and so it is possible that a test in one example could be misread from the digitized test order as the other test in that example. However, by comparing the candidate test order to the disease area group (provided in the context data) the misread test order may be rejected and, optionally, the correct test order may be suggested as an alternative. For example, in example i above, the candidate test order may include HCV. However, the context data may indicate that the disease area group is fertility which is not associated with HCV. This mis-match can trigger a rejection of the candidate test order. The system can then suggest a test for hCG as an alternative candidate test order since this is a test order with a similar spelling to HCV which is in the fertility disease area group.

In further examples, the context data may include a sample tube type and sample additives. For example, this may include cap color and tube dimensions which can be used to process and register samples in the automated laboratory 30. The data processing agent 10 may be configured to compare the candidate test order to the tube type so that only test orders (assays) which are not intended for the sample tube type are rejected. Typically, automated laboratory systems may check if a sample tube type corresponds to a test order, rather than the opposite, wherein the candidate test order is verified as corresponding to the sample tube type.

For example, D-Dimer test cannot be made with serum, plasma is needed. Either citrate (ACN 20530) or heparin/EDTA plasma (ACN 20531). The compatibility of the candidate test order with a sample/tube type and sample attributes can be derived from one or more of: Guidelines / best practices as e.g. CLSI GP419, IVD vendor method sheets, and/or Laboratory requirements.

Additionally, the verification of the candidate test order may also consider the availability of the candidate test order in a particular target laboratory.

In further examples, where multiple analytical results are received in respect of a same test order, e.g., from multiple different laboratories or from multiple different analyzers, then the context data may include those analytical results. In these examples, a test order may be associated with multiple samples and/or specify multiple analytical tests. Where the results for a first one of the samples or analytical tests are received before a second of the analytical tests has been performed, then the results may be used to verify the second analytical test specified in the test order. In this way, the combination of all the analytical tests ordered in one test order and the results of those tests may form part of the context data for verifying the candidate test order. For example, where blood for a first tube A and for a second tube B are obtained from one patient and analytical results associated with tube B are received before the test order in respect of tube A has been verified (for example, if tube A was sent to a different laboratory which is further away than the laboratory for tube B) then the results for tube B may form part of the context data for verifying the test order in respect of tube A.

Additionally, where multiple test orders are received for one sample and/or a patient, then the verification of each candidate test order for those multiple test orders may include assessing the combination of the multiple test orders. In this way, the combination of all the tests ordered for one patient and/or samples thus form part of the context data. Where a combination of multiple tests are determined to be unlikely to be ordered together, then one or more of the candidate test orders estimated from the multiple test orders may be rejected.

In step S214 if the data processing agent 10 rejects the candidate test order, the data processing agent 10 may provide an indication to a user such as the person who provided the digitized medical test order, a patient, or a lab operator. The data processing agent 10 may also provide (e.g., on a user interface, in an email etc) a prompt to perform a follow up action such as collecting another sample and/or placing the medical test order again. In some examples, if the candidate test order was rejected, the candidate test order may still be transmitted to the automated laboratory 30 (as being the closest match to the estimated text on the digitized test order). Additionally or alternatively, in this example, the data processing agent 10 may determine and propose an alternative test order to the automated laboratory 30 which corresponds to a more plausible test order (e.g., according to the context data discussed above).

For example:
i. Candidate test order derived from the estimated text is Glucose measured in urine (Roche ID: GLUC3U, ACN: 20631, urine) but the delivered and received sample material is NaF EDTA (sodium fluoride as glycolysis inhibitor) stabilized blood. Therefore the verification step detects a mismatch between the sample type and the sample type associated with the candidate test order. The data processing agent 10 changes the order to GLUC3, ACN: 20630 (serum/plasma). In addition, a notification of the change is provided to a user.
ii. The candidate test order derived from the estimated text relates to a test which has already expired for the associated sample because the duration from sampling time point to current time is greater than the analyte stability specified in the database. Therefore, potential replacement test orders with longer associated analyte stability may be proposed instead. For example, stability in blood at room temperature is given in the following examples, for a few analyte examples.
   a. Cardiac
      (LDL: 1d; HDL: 2d; Cholesterol: 2-7d; Apolipoproteins Al, All, B: 36h 4-8°C;
      Homocysteine: 1h, 6h 2-6°C; CRP: 3w (2-6°C)); candidate test order: Homocysteine changed to LDL and HDL assays.
   b. Hepatology
      LDH: 1h; Alkaline phosphatase (ALP): 4d; NH3: 15min in EDTA; AST: 7d; candidate test order: LDH changed to Alkaline phosphatase (ALP) assay.
   c. Inflammation, Hepatology
      GLU: 10min, 2h** EDTA, citrate, fluoride tubes; TP: total protein 1d; HbA1c: 3 d (EDTA-blood, LOINC: 59261-8); candidate test order: GLU changed to total protein (TP) and HbA1c assay.
   d. Coagulation
      (anti-thrombin and Willebrand-factor <48h; PT and factors II, V, VII, IX, XI and XII < 6h; aPTT, fibrinogen and factor VIII: <3h)⁵; The candidate test order is coagulation factor VIII, with a time from sampling to analysis limit of 3h (page 274)⁶. The duration since sampling already exceeds 3h. However, testing is realistic within 6h. Coagulation factor V, XIII and partial thromboplastin time (aPTT, stability 1 day @RT) and protein C (1 week stability at RT, sodium citrate plasma) can be measured as an alternative. Additionally, a request may be sent out for a new sample with shorter turn-a-round time for the factor VIII determination.
iii. If interferences associated with the candidate test order exceed a certain limit, then a test with a higher limit may be suggested. For example, the candidate test order derived form the candidate test may be Creatine Kinase MB Serum/Plasma STAT on Abbott Alinity i, with product code 04V3820. However, an interference check shows that a short-term previous sample (measured only hours ago) had high I-Index (interference-index) (Icterus/Bilirubin) above the assay specific limit of 20.0mg/dL. Therefore, as alternative, a Roche Assay for the same analyte with higher interference limit may be proposed as replacement: PN: 07027087190 with a limit of 34.0mg/dL.
⁵ Pre-Examination procedures in laboratory diagnostics, Walter G. Guder et all, 2015, p. 274 ⁶ Pre-Examination procedures in laboratory diagnostics, Walter G. Guder et all, 2015

However, in further examples, when the candidate test order is rejected, the data processing agent 10 may be configured to inform the user of the likely error without proposing alternative tests. A troubleshooter may contact the provider of the digitized test order (e.g., a doctor or clinic) to request a new order.

In some examples, the actions taken by the data processing agent 10 in the event of a candidate test order being rejected may be configurable. For example, a user may configure a level of the interaction, from cautious wherein the data processing agent 10 simply provides warnings or error report without altering the candidate test orders to less cautious wherein the data processing agent 10 is configured to change the candidate test orders automatically to more plausible matches while providing only silent notifications about said changes.

By verifying and optionally correcting the candidate test order using context data, the translation of digitized test orders in to machine-readable test orders is more reliable, without requiring human interference. The workflow, efficiency, and patient outcomes of the automated laboratory system 1 are therefore improved.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### References

A number of publications are cited above in order to more fully describe and disclose the invention and the state of the art to which the invention pertains. Full citations for these references are provided below. The entirety of each of these references is incorporated herein.
[1] The impact of the implementation of electronic ordering on hospital pathology services, A. Georgiou et al, 2012, DOI: http://dx.doi.org/https://doi.org/10.26190/unsworks/35
[2] Clinical Laboratory Diagnostics, Prof. Dr. med, Lothar Thomas, 01.06.2023, www.clinical-laboratory-diagnostics.com, chapter 4 practical examples, page 66
[3] Laboratory Diagnostics Pathways, W. Hofmann, J. Aufenanger, G. Hoffmann, De Gruyter, 2nd edition 2016, ISBN: 987-3-11-045367-6, chapter 4

## Claims

1. A computer-implemented method of processing a sample based on a digitized medical test order, the computer-implemented method comprising the following steps:
receiving, at a data processing agent, the digitized medical test order, the digitized medical test order comprising an image or a sound file comprising instructions for processing the sample;
estimating, by the data processing agent, candidate text from the digitized medical test order by performing character recognition on the image or speech recognition on the sound file;
comparing, by the data processing agent, the estimated candidate text to a set of predetermined test specifications to propose a candidate test order being requested by the digitized medical test order;
verifying, by the data processing agent, the candidate test order utilising context data associated with the digitized medical test order; and
when the candidate test order is successfully verified, transmitting, by the data processing agent, the verified candidate test order to a control unit of an automated laboratory, wherein said control unit is configured to match the sample to the verified candidate test order and instruct an automated analyzer of the automated laboratory to process said sample according to the verified candidate test order to obtain a test result.

2. The computer-implemented method of claim 1 wherein, when the candidate test order is not successfully verified, the method further comprises rejecting the digitized medical test order.

3. The computer-implemented method of claim 2 wherein the rejecting the digitized medical test order comprises generating an indication for a user interface that the digitized medical test order is rejected.

4. The computer-implemented method of claims 2 or 3 wherein, when the candidate test order is rejected, the method further comprises indicating an alternative candidate test order, wherein the alternative candidate test order is generated based on the context data and/or the estimated candidate text.

5. The computer-implemented method of any preceding claim wherein:
the set of predetermined test specifications have associated synonyms and test parameters, and
comparing the estimated candidate text to the set of predetermined test specifications comprises comparing the estimated candidate text to the associated synonyms and test parameters associated with each predetermined test specification.

6. The computer-implemented method of claim 5 wherein
proposing the candidate test order comprises determining a subset of the predetermined test specifications representing the closest matches between the estimated candidate text and the associated synonyms and/or test parameters, and
selecting a most likely of the predetermined test specifications in the subset as the candidate test order.

7. The computer-implemented method of any preceding claim wherein:
the set of predetermined test specifications are formatted as industry test codes having associated synonyms, test options and/or test parameters, and
proposing the candidate test order comprises mapping the estimated candidate text to one of the industry test codes.

8. The computer-implemented method of any preceding claim wherein the context data comprises one or more from a group containing: patient data, patient test order history, patient demographic information, clinic location of origin of the digitized medical test order, clinic test order history, target laboratory for the digitized medical test order, target laboratory test availability, identity of a physician submitting the medical test order, sample type associated with the digitized medical test order, and/or additional digitized medical test orders submitted in combination with the digitized medical test order.

9. The computer-implemented method of any preceding claim wherein
verifying the candidate test order comprises performing a binary determination of whether the candidate test order is compatible with the context data and rejecting the digitized medical test order if the candidate test order is incompatible.

10. The computer-implemented method of any preceding claim wherein verifying the candidate test order comprises:
using the context data to determine a likelihood of the candidate test order being correct;
comparing the likelihood to a configurable threshold; and
successfully verifying or rejecting the candidate test order depending on if the likelihood is above the threshold.

11. The computer-implemented method according to any preceding claim wherein the context data comprises clinic and/or patient history information, the clinic being a source location of the digitized medical test order, and
verifying the candidate test order comprises determining a likelihood of the candidate test order being requested based on the clinic and/or patient history information.

12. The computer-implemented method according to any preceding claim wherein
the context data comprises a clinic and/or physician medical specialism(s) associated with the digitized test order, the clinic and/or physician being a source location of the digitized medical test order, and
verifying the candidate test order comprises determining a likelihood of the candidate test order being requested in relation to that medical specialism.

13. The computer-implemented method of any preceding claim wherein verifying the candidate test order comprises:
retrieving test parameters associated with the candidate test order, and
comparing the test parameters to the context data, and
determining if the context data corresponds to the test parameters.

14. The computer-implemented method of claim 12 wherein
the context data comprises patient demographic data for a patient associated with the digitized medical test order, and
the test parameters comprise expected patient demographics associated with the candidate test order,
wherein verifying the candidate test order comprises determining if the patient demographic data corresponds to the expected patient demographics.

15. A data processing agent for processing a digitized medical test order, wherein the data processing agent comprises:
a test order input module configured to receive a digitized medical test order, the digitized medical test order comprising an image or a sound file comprising instructions processing a sample,
a text generation module configured to estimate candidate text from the digitized medical test order by performing character recognition on the image or speech recognition on the sound file;
a text comparison module configured to compare the estimated candidate text to a set of predetermined test specifications to propose a candidate test order being requested by the digitized test order; and
an order verification module configured to verify the candidate test order utilising context data associated with the digitized medical test order; and
an instruction transmission module configured to, when the candidate test order is successfully verified, transmit the verified candidate test order to a control unit of an automated laboratory for instructing an automated analyzer to process said sample according to the verified candidate test order.
